# EUROPÄISCHE PATENTSCHRIFT

(11) **EP 3 808 288 B1**
(45) Veröffentlichungstag und Bekanntmachung des Hinweises auf die Patenterteilung: **04.01.2023**
(21) Anmeldenummer: 20201314.0
(22) Anmeldetag: 12.10.2020
(51) Int. Cl.: A61B 17/22, A61B 17/00

(54) **LITHOTRIPSIEVORRICHTUNG**
LITHOTRIPSY DEVICE
DISPOSITIF DE LITHOTRIPSIE

(30) Priorität: 17.10.2019 DE 102019128043; 17.10.2019 DE 102019128044
(43) Veröffentlichungstag der Anmeldung: 21.04.2021
(73) Patentinhaber: Karl Storz SE & Co. KG, 78532 Tuttlingen (DE)
(72) Erfinder: Staud, Ralf, 78532 Tuttlingen (DE); Glöggler, Bernhard, 78532 Tuttlingen (DE); Willunat, Annika, 78532 Tuttlingen (DE)

(56) Entgegenhaltungen:
- DE-A1- 3 927 260
- DE-U1-202010 001 176
- JP-A- H05 309 096
- US-A1- 2010 256 535
- US-A1- 2013 255 837
- US-A1- 2019 150 703

## Beschreibung

### Stand der Technik

Die Erfindung betrifft eine Lithotripsievorrichtung, insbesondere eine intrakorporale Lithotripsievorrichtung, gemäß Anspruch 1, sowie einem Verfahren zum nicht-therapeutischen und nicht-chirurgischen Betrieb einer solchen Lithotripsievorrichtung gemäß Anspruch 20.

Aus der US 6,875,220 B2 ist bereits eine Lithotripsievorrichtung bekannt, welche eine Zertrümmerungseinheit umfasst, wobei die Zertrümmerungseinheit zur Beseitigung von Konkrementen eingerichteten ist und wenigstens eine zur Beaufschlagung von Konkrementen eingerichtete Sonde, sowie wenigstens eine zumindest mittelbar Impulse auf die Sonde übertragende Impulseinheit umfasst.

Aus der DE 39 27 260 A1, der US 2010/0256535 A1, der DE 20 2010 001 176 U1 und der JP H05 309096 A geht im Zusammenhang mit extrakorporalen Lithotriptern hervor, dass eine Zertrümmerungseinheit eines solchen Lithotripters zumindest teilweise aus einer Keramik, beispielsweise Zirkonoxid, Siliziumnitrid oder Siliziumoxid, bestehen kann.

Die US 2019/150703 A1 offenbart einen intrakorporale Lithotripter mit einer Zertrümmerungseinheit, welche eine Sonotrode umfasst, welche einen auf amorphen Kohlenstoff basierten Werkstoff aufweist.

Aus der US 2013/0255837 A1 sind allgemein verschiedene Zusammensetzungen und Herstellungsverfahren metallischer Gläser bekannt.

Die Aufgabe der Erfindung besteht insbesondere darin, eine gattungsgemäße Vorrichtung mit verbesserten Eigenschaften hinsichtlich einer Effizienz, insbesondere einer Energieeffizienz und/oder einer Bauteileffizienz, bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Lithotripsievorrichtung, insbesondere einer intrakorporalen Lithotripsievorrichtung, mit einer Zertrümmerungseinheit, welche zur Beseitigung von Konkrementen eingerichteten ist und welche wenigstens eine zur wenigstens mittelbaren Beaufschlagung von Konkrementen eingerichtete Sonde, sowie wenigstens eine zumindest mittelbar Impulse auf die Sonde übertragende Impulseinheit umfasst, welche zur wenigstens mittelbaren Beaufschlagung von Konkrementen einen Sondenstab, sowie zur Aufnahme von Impulsen einen Sondenkopf aufweist.

Ferner geht die Erfindung davon aus, dass die Zertrümmerungseinheit wenigstens teilweise, insbesondere zumindest zu einem Großteil und besonders bevorzugt vollständig, aus einem amorphen Werkstoff besteht, welcher eine Elastizitätsgrenze von wenigstens 1,5% und/oder eine Festigkeit von wenigstens 500 MPa aufweist. Hierdurch kann insbesondere eine Effizienz verbessert werden. Vorteilhaft kann die Nutzung des amorphen Werkstoffs eine Energieübertragung zwischen Bauteilen der Lithotripsievorrichtung und/oder auf die Konkremente verbessert werden, wodurch eine Verbesserung der Energieeffizienz im Betrieb der Lithotripsievorrichtung erzielt werden kann. Weiter vorteilhaft kann durch die Nutzung des amorphen Werkstoffs Abnutzungserscheinungen verringert und/oder eine Standzeit von Komponenten und Bauteilen der Lithotripsievorrichtung erhöht werden, wodurch eine Verbesserung einer Bauteileffizienz erzielt werden kann. Insgesamt kann somit insbesondere gleichzeitig eine Energieeffizienz als auch eine Bauteileffizienz verbessert werden.

Unter einer "Lithotripsievorrichtung" soll insbesondere ein, vorzugsweise funktionsfähiger Bestandteil, insbesondere eine Unterbaugruppe und/oder eine Konstruktions- und/oder eine Funktionskomponente eines Lithotripters verstanden werden. Vorzugsweise kann die Lithotripsievorrichtung den Lithotripter zumindest teilweise, vorzugsweise zumindest zu einem Großteil und besonders bevorzugt vollständig ausbilden. Die Lithotripsievorrichtung ist zu einer Zertrümmerung von Konkrementen eingerichtet. Unter "Konkrementen" sollen insbesondere sich in Körpern durch Abscheidung entstehende feste Gebilde verstanden werden, welche zumindest teilweise, vorzugsweise zumindest zu einem Großteil und besonders bevorzugt vollständig aus Salzen bestehen, wie beispielsweise Nierensteinen, Gallensteinen, Harnsteinen, Speichelsteinen oder dergleichen. Die Lithotripsievorrichtung ist vorzugsweise als eine intrakorporale Lithotripsievorrichtung ausgebildet, welche dazu ausgebildet ist, zumindest teilweise und vorzugsweise zumindest zu einem Großteil in eine insbesondere künstliche und/oder natürliche Öffnung, insbesondere Körperöffnung, eingeführt zu werden, um dort angesammelte Konkremente zu zertrümmern und/oder zu entfernen. Unter "eingerichtet" soll insbesondere speziell programmiert, ausgelegt, ausgebildet und/oder ausgestattet verstanden werden. Darunter, dass ein Bauteil zu einer bestimmten Funktion eingerichtet ist, soll insbesondere verstanden werden, dass das Bauteil diese bestimmte Funktion in zumindest einem Anwendungs- und/oder Betriebszustand erfüllt und/oder ausführt. Unter dem Ausdruck "zumindest zu einem Großteil" soll dabei insbesondere zumindest zu 55 %, vorzugsweise zumindest zu 65 %, bevorzugt zumindest zu 75 %, besonders bevorzugt zumindest zu 85 % und ganz besonders bevorzugt zumindest zu 95 %, sowie vorteilhaft vollständig verstanden werden und zwar insbesondere mit Bezug auf ein Volumen und/oder eine Masse eines Bauteils. Unter einer "Zertrümmerungseinheit", soll insbesondere eine Einheit verstanden werden, welche sich wenigstens aus der insbesondere intrakorporalen Sonde und der Impulseinheit zusammensetzt. Die Zertrümmerungseinheit kann ferner weitere Einheiten, Komponenten und Bauteile aufweisen. Unter einer "Sonde" soll insbesondere ein stab-, röhren- und/oder schlauchförmiges Bauteil verstanden werden, welches zur Untersuchung und/oder Behandlung eingerichtet ist. Vorzugsweise ist die Sonde als eine intrakorporale Sonde ausgebildet. Unter einer "intrakorporalen Sonde" soll insbesondere eine Sonde, und insbesondere ein Teil der Lithotripsievorrichtung verstanden werden, welche/welcher dazu eingerichtet ist, zumindest teilweise, vorzugsweise zumindest zu einem Großteil und besonders bevorzugt vollständig, in eine insbesondere künstliche und/oder natürliche Öffnung, insbesondere Körperöffnung, eingeführt zu werden, um beispielsweise dort angesammelte Konkremente zu zertrümmern und/oder zu entfernen. Die Sonde weist insbesondere zumindest eine 7 auf, welche dazu eingerichtet ist Konkremente unmittelbar zu kontaktieren, sowie insbesondere Impulse auf diese zu übertragen. Ferner weist die Sonde insbesondere wenigstens einen Sondenkopf auf, welcher zur Aufnahme von den von der Impulseinheit bereitgestellten Impulsen eingerichtet ist. Zudem weist die Sonde insbesondere einen Sondenstab auf, welcher aufgenommene Impulse von dem Sondenkopf zur Sondenspitze zumindest mittelbar überträgt. Unter "zumindest mittelbar" soll insbesondere indirekt aber vorzugsweise auch direkt, also insbesondere unmittelbar, verstanden werden. Vorzugsweise ist der Sondenkopf von dem Sondenstab separat ausgebildet. Es ist jedoch auch denkbar, dass die Sonde zumindest teilweise, vorzugsweise zumindest zu einem Großteil und besonders bevorzugt vollständig einstückig ausgebildet ist. Darunter, dass "ein Bauteil und ein weiteres Bauteil zumindest teilweise einteilig ausgebildet/verbunden sind", soll insbesondere verstanden werden, dass zumindest ein Element und/oder Teil des Bauteils und zumindest ein Element und/oder Teil des weiteren Bauteils einteilig ausgebildet/verbunden sind. Unter "einstückig" soll insbesondere zumindest stoffschlüssig verbunden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Beispielsweise könnten die Sondenspitze und der Sondenstab einstückig ausgebildet sein. Unter "Impulsen" sollen insbesondere Stoßimpulse und/oder Schwingungsimpulse verstanden werden. Unter einer "Impulseinheit" soll insbesondere eine Einheit verstanden werden, welche zu übertragende Impulse erzeugt und/oder bereitstellt. Unter einem "amorphen Werkstoff" soll insbesondere ein Werkstoff verstanden werden, welcher eine unregelmäßige, vorzugsweise nicht kristalline, Anordnung von Atomen aufweist und welche vorteilhaft lediglich über Nahordnung, nicht aber Fernordnung verfügt. Der amorphe Werkstoff weist insbesondere eine Elastizitätsgrenze von wenigstens 1,5%, vorzugsweise von wenigstens 1,75% und besonders bevorzugt von wenigstens 2% und/oder insbesondere von höchstens 2,75%, vorzugsweise von höchstens 2,5 und besonders bevorzugt von höchstens 2,25% auf. Ganz besonders bevorzugt weist der amorphe Werkstoff eine Elastizitätsgrenze von zumindest im Wesentlichen 3% auf. Unter "zumindest im Wesentlichen" in Bezug auf einen Wert einer Wertangabe soll insbesondere verstanden werden, dass der Wert im Rahmen einer Ungenauigkeit von höchstens 15 %, vorzugsweise höchstens 5 % und besonders bevorzugt von höchstens 1 % genau ist oder aber auch frei von einer Ungenauigkeit ist, also vorzugsweise genau ist. Der amorph Werkstoff weist insbesondere eine Festigkeit von wenigstens 500 MPa, vorzugswiese von wenigstens 1000 MPa und besonders bevorzugt von wenigstens 1500 MPa und/oder insbesondere von höchstens 3400 MPa, vorzugsweise von höchstens 2900 MPa und besonders bevorzugt von höchstens 2400 MPa auf. Ganz besonders bevorzugt beträgt eine Festigkeit des amorphen Werkstoffs zumindest im Wesentlichen 2000 MPa. Beispielsweise beträgt ein Verhältnis aus der Elastizitätsgrenze und der Festigkeit des amorphen Werkstoffs wenigstens 400 MPa/%, vorzugsweise wenigstens 600 MPa/% und besonders bevorzugt wenigstens 800 MPa/% und/oder höchstens 1600 MPa/%, vorzugsweise höchstens 1400 MPa/% und besonders bevorzugt höchstens 1200 MPa/%. Ganz besonders bevorzugt beträgt ein Verhältnis der Festigkeit und der Elastizitätsgrenze zumindest im Wesentlichen 1000 MPa/%.

Beispielsweise sind Hochleistungskunststoffe als amorphe Werkstoffe in der Lage angefordert hohe Elastizitätsgrenzen zu erzielen. Auch sind Hochleistungsmetalllegierungen in der Lage, entsprechend hohe Festigkeiten zu erzielen. Um jedoch gleichzeitig eine derartige Elastizitätsgrenze und Festigkeit in Kombination zu erzielen, wird erfindungsgemäß vorgeschlagen, dass der amorphe Werkstoff metallisches Glas ist. Es kann vorteilhaft eine Effizienz weiter verbessert werden. Insbesondere weisen metallische Gläser der Anforderung entsprechend hohe Elastizitätsgrenzen auf, welche eine elastische Energieübertragung und somit eine Energieeffizient verbessert. Ferner weisen metallische Gläser der Anforderung entsprechend eine hohe Festigkeit auf, welche die Standzeit und somit die Bauteileffizienz erhöht. Unter "metallischem Glas" soll insbesondere ein Werkstoff verstanden werden, welcher wenigstens ein Metall umfasst, welches in einer amorphen Struktur vorliegt. Auch soll unter metallischem Glas insbesondere amorphes Metall verstanden werden. Das metallische Glas ist vorzugsweise eine Legierung aus Metallen oder Nichtmetallen und Metallen.

Weiter wird vorgeschlagen, dass der amorphe Werkstoff wenigstens ein Metall, wenigstens ein Übergangsmetall, wenigstens ein Erdalkalimetall und/oder ein Gemisch dieser umfasst. Es kann vorteilhaft eine Festigkeit und/oder Elastizitätsgrenze weiter erhöht werden, wodurch eine Effizienz weiter verbessert werden kann. Bei dem Metall kann es sich insbesondere um Kupfer, Aluminium und/oder dergleichen handeln. Ferner kann es sich bei den Übergangsmetallen um Zirconium, Niob, Titan, Nickel und/oder dergleichen handeln. Ferner kann es sich bei dem Erdalkalimetall um Beryllium, Magnesium und/oder der gleichen handeln. Insbesondere ist der amorphe Werkstoff eine Legierung aus Metallen, Übergangsmetallen und/oder Erdalkalimetallen. Besonders bevorzugt handelt es sich bei dem amorphe Werkstoff um ein metallisches Glas, insbesondere Vitreloy^{™} 1, Vitreloy^{™} 1b, Vitreloy^{™} 4, Vitreloy^{™} 101, Vitreloy^{™} 105, Vitreloy^{™} 106, Vitreloy^{™} 106a, und/oder AMZ^{™} 4 bekannt. Das amorphe Material weist dabei beispielsweise eine der folgenden Zusammensetzungen auf:

| | |
|---|---|
| Vitreloy^{™} 1: | Zr_{41,2}Be_{22,5}Ti_{13,8}Cu_{12,5}Ni₁₀ (at%) |
| Vitreloy^{™} 1b: | Zr₄₄Be₂₅Ti₁₁Cu₁₀Ni₁₀ (at%) |
| Vitreloy^{™} 4: | Zr_{46,75}Ti_{8;25}Cu_{7,5}Ni₁₀Be_{27,5} (at%) |
| Vitreloy^{™} 101: | Cu₄₇Ti₃₄Zr₁₁Ni₈ (at%) |
| Vitreloy^{™} 105: | Zr_{52,5}CU_{17,9}Ni_{14,6}Al₁₀Ti₅ (at%) |
| Vitreloy^{™} 106: | Zr₅₇Cu_{15,4}Ni_{12,6}Al₁₀Nb₅ (at%) |
| Vitreloy^{™} 106a: | Zr_{58,5}Cu_{15,6}Ni_{12,8}Al_{10,3}Nb_{2,8} (at%) |
| AMZ^{™} 4: | Zr_{59,3}Cu_{28,8}Al_{10,4}Nb_{1,5} (at%) |

Der amorphe Werkstoff könnte Kupfer und/oder Titan basiert sein. Darunter, dass der amorphe Werkstoff "auf einem chemischen Element basiert" soll insbesondere verstanden werden, dass dieses chemische Element einen größten Anteil des amorphen Werkstoff ausmacht. Da insbesondere Zirconium basierte amorphe Werkstoffe den Anforderung entsprechend hoher Festigkeiten und Elastizitätsgrenzen aufweisen wird, um vorteilhaft eine Effizienz weiter zu erhöhen in einer bevorzugten Ausgestaltung der Erfindung vorgeschlagen, dass der vorbenannte amorphe Werkstoff Zirconium basiert ist. Beispielsweise bildet Zirconium den größten Anteil bei den amorphen Werkstoffen Vitreloy^{™} 106 oder AMZ^{™} 4 aus. Insbesondere besteht der amorphe Werkstoff wenigstens zu 40%, vorzugsweise zu wenigstens 45%, besonders bevorzugt zu wenigstens 50% und ganz besonders bevorzugt zu wenigstens 55% aus Zirconium. Besonders bevorzugt ist der amorphe Werkstoff frei von Beryllium und/oder Niob, wodurch vorteilhaft eine Biokompatibilität des amorphen Werkstoffs verbessert werden kann.

Es wird weiterhin vorgeschlagen, dass die Sonde wenigstens teilweise, insbesondere zumindest zu einem Großteil und besonders bevorzugt vollständig, aus dem amorphen Werkstoff besteht. Es kann vorteilhaft eine Energieeffizienz weiter erhöht werden. Insbesondere kann eine Energieübertragung zwischen Projektil und Sonde verbessert werden. Vorzugsweise ist die Sonde mehrteilig. Besonders bevorzugt besteht die Sonde höchstens teilweise, also beispielsweise stets weniger als vollständig, aus dem amorphen Werkstoff, wodurch vorteilhaft Kosten eingespart werden können.

Weiter wird vorgeschlagen, dass der zur Aufnahme von Impulsen eingerichtete Sondenkopf zumindest teilweise, insbesondere zumindest zu einem Großteil und besonders bevorzugt vollständig, aus dem amorphen Werkstoff besteht. Es kann vorteilhaft eine Energieeffizienz noch weiter erhöht werden. Insbesondere kann eine Energieübertragung zwischen Projektil und Sonde weiter verbessert werden. Vorzugsweise ist der Sondenkopf einstückig ausgebildet.

Des Weiteren wird vorgeschlagen, dass der Sondenstab frei von dem amorphen Werkstoff ist. Es kann vorteilhaft eine Effizienz erhöht werden. Insbesondere kann eine Bauteileffizienz erhöht werden, da mit dem Sondenstab separat aus einem im Vergleich zum amorphen Werkstoff günstigeren Material, wie beispielsweise Stahl, Titan oder dergleichen hergestellt werden kann. Insbesondere kann vermieden werden, dass ein Teil der Sonde, welcher mit einem Patienten in Berührung kommt aus einem nicht biokompatiblen Material besteht. Ferner ist insbesondere die Sondenspitze der Sonde frei von dem amorphen Werkstoff.

In einem weiteren Aspekt der Erfindung, welcher insbesondere für sich alleine genommen und/oder in Kombination mit weiteren Aspekten der Erfindung betrachtet werden kann, wird vorgeschlagen, dass der Sondenstab und der Sondenkopf form- und/oder kraftschlüssig, insbesondere reibschlüssig, miteinander verbunden sind. Indem eine effiziente Verbindung von Sondenstab und Sondenkopf hergestellt wird, kann vorteilhaft eine Energieübertragung zwischen diesen Bauteilen weiter verbessert werden. Weiter vorteilhaft kann ein ungewolltes Auslösen der Bauteile vermieden werden, wodurch insbesondere eine Standzeit und somit eine Bauteileffizienz verbessert werden kann. Besonders vorteilhaft kann eine Effizienz einer Herstellung und/oder Montage verbessert werden bzw. eine Herstellung und/oder Montage vereinfacht werden. Unter einer "form- und/oder kraftschlüssigen Verbindung" ist insbesondere zu verstehen, verbunden, vorzugsweise lösbar verbunden zu sein, wobei eine Haltekraft zwischen zwei Bauteilen vorzugsweise über einen geometrischen Eingriff der Bauteile ineinander und/oder über eine Reibungskraft, die vorzugsweise zwischen den Bauteilen wirkt, übertragen wird.

Außerdem wird zudem vorgeschlagen, dass die Sonde einen Eingriffsbereich aufweist, in welchem der Sondenstab und der Sondenkopf miteinander in Eingriff stehen. Durch Schaffung einer festen Verbindung der Bauteile kann eine Energieeffizienz und/oder eine Bauteileffizienz weiter verbessert werden. Insbesondere kann eine Herstellung und/oder Montage vereinfacht werden. Unter einem "Eingriff soll insbesondere verstanden werden, dass ein Bauteil ein anderes Bauteil von wenigstens drei Seiten umschließt. Unter einer "Seite" soll insbesondere eine gedachte Ebene verstanden werden, welche senkrecht zu einer Koordinatenachse, insbesondere einer Abszissenachse, einer Ordinatenachse und /oder einer Applikatenachse, orientiert ist. Demnach kann ein Bauteil in einem dreidimensionalen Koordinatensystem maximal von sechs Seiten umgeben sein. Denkbar ist, dass sich der Eingriffsbereich einer gesamten Haupterstreckung des Sondenkopfs entspricht. Vorteilhaft ist eine Erstreckung des Eingriffsbereichs jedoch kleiner als eine Haupterstreckung des Sondenkopfs. Unter einer "Haupterstreckung" eines Bauteils soll dabei insbesondere eine längste Erstreckung des Bauteils entlang dessen Haupterstreckungsrichtung verstanden werden. Unter einer "Haupterstreckungsrichtung" eines Bauteils soll dabei insbesondere eine Richtung verstanden werden, welche parallel zu einer längsten Kante eines kleinsten gedachten Quaders verläuft, welcher das Bauteil gerade noch vollständig umschließt. Der Eingriffsbereich ist insbesondere zumindest teilweise von einem Eingriffsabschnitt des Sondenstabs gebildet. Ferner ist der Eingriffsbereich zumindest teilweise von einem Eingriffsabschnitt des Sondenstabs gebildet. Vorzugsweise kann es sich bei der 6 um eine Vollausnehmung handeln.

Um insbesondere eine stabile und energieeffiziente Energieübertragung zu erzielen, kann vorteilhaft eine Verbindung zwischen Sondenkopf und Sondenstab weiter verbessert werden, indem, wie in einer bevorzugten Ausgestaltung der Erfindung vorgeschlagen, der Eingriffsbereich sich über wenigstens 3 mm, vorzugsweise wenigstens 3,5 mm und besonders bevorzugt wenigstens 4 mm, entlang einer Haupterstreckungsachse des Sondenstab und/oder des Sondenkopfs erstreckt. Ferner erstreckt sich der Eingriffsbereich insbesondere über höchstens 7 mm, vorzugsweise über höchstens 6 mm und besonders bevorzugt über höchstens 5 mm, entlang einer Haupterstreckungsachse des Sondenstab und/oder des Sondenkopfs. Unter einer "Haupterstreckungsachse" soll insbesondere eine Achse verstanden werden, welche parallel zu einer Haupterstreckungsrichtung des Bauteils und insbesondere durch dessen Massen- und/oder geometrischen Mittelpunkt verläuft. Vorzugsweise ist die Erstreckung des Eingriffsbereichs wesentlich kleiner als eine Haupterstreckung des Sondenstab und/oder des Sondenkopfs. Unter "wesentlich kleiner" soll hier insbesondere weniger als die Hälfte verstanden werden.

Es ist denkbar, dass der Sondenstab den Sondenkopf im Eingriffsbereich umgeben könnte, beispielsweise wenn der Sondenstab über eine Verbreiterung verfügen würde. In einer besonders bevorzugten Ausgestaltung der Erfindung wird jedoch vorgeschlagen, dass im Eingriffsbereich der Sondenkopf den Sondenstab umgibt. Es kann vorteilhaft eine Energieeffizienz und/oder eine Bauteileffizienz weiter verbessert werden. Insbesondere kann eine Herstellung und/oder Montage weiter vereinfacht werden. Insbesondere umgibt der Sondenkopf den Sondenstab von wenigstens vier Seiten. Besonders bevorzugt umgibt der Sondenkopf den Sondenstab von fünf Seiten.

Ferner wird vorgeschlagen, dass im Eingriffsbereich der Sondenkopf und/oder der Sondenstab profiliert sind. Es kann vorteilhaft eine Energieeffizienz und/oder eine Bauteileffizienz besonders verbessert werden. Insbesondere kann entweder der Sondenkopf oder der Sondenstab profiliert sein. Ferner ist denkbar, dass sowohl der Sondenkopf als auch der Sondenstab profiliert sind. Darunter, dass ein Bauteil profiliert ist" soll insbesondere verstanden werden, dass dieses bereichsweise mit einer Profilierung versehen ist. Vorzugsweise weist die Profilierung eine Undulation, ein Gewinde Einkerbungen, Vorsprünge und/oder dergleichen auf.

Eine Energieeffizienz und/oder eine Bauteileffizienz kann ganz besonders verbessert werden, wenn der Sondenkopf und der Sondenstab, wie vorgeschlagen, miteinander verpresst, insbesondere vercrimpt, sind. Insbesondere kann eine Herstellung und/oder Montage vereinfacht werden. Vorzugsweise wird der Sondenkopf und/oder der Sondenstab durch verformen miteinander verpresst. Insbesondere ist der Sondenkopf radial mit dem Sondenstab verpresst, insbesondere vercrimpt. Vorzugsweise schneiden im Eingriffsbereich insbesondere ein profilierter Anteil des Sondenkopfs und/oder des Sondenstabs ineinander ein.

In einem weiteren Aspekt der Erfindung, welcher insbesondere für sich alleine genommen und/oder in Kombination mit weiteren Aspekten der Erfindung betrachtet werden kann, wird vorgeschlagen, dass der Sondenkopf und der Sondenstab miteinander stoffschlüssig verbunden sind. Es kann vorteilhaft eine Energieeffizienz und/oder eine Bauteileffizienz weiter verbessert werden. Unter "stoffschlüssig verbunden" soll insbesondere verstanden werden, dass die Objekte durch atomare oder molekulare Kräfte zusammengehalten werden, wie beispielsweise beim Löten, Schweißen, Kleben und/oder Vulkanisieren.

In einer Ausgestaltung der Erfindung sind der Sondenkopf und der Sondenstab miteinander verschweißt. In der gleichen oder einer alternativen Ausgestaltung der Erfindung sind der Sondenkopf und der Sondenstab adhäsiv miteinander verbunden. Es kann vorteilhaft eine besonders effiziente Verbindung geschaffen werden.

Weiterhin wird vorgeschlagen, dass der Sondenkopf und der Sondenstab miteinander umspritzt sind. Es kann vorteilhaft eine effiziente Verbindung geschaffen werden. Insbesondere kann eine Herstellung und/oder Montage vereinfacht werden. Insbesondere ist der Sondenstab von dem Sondenkopf umspritzt. Vorzugsweise bilden Sondenstab und Sondenkopf zusammen eine Mehrkomponenten-Spritzgussbaugruppe aus.

Außerdem wird vorgeschlagen, dass die Impulseinheit wenigstens eine Stoßeinheit umfasst, welche zur Bereitstellung von Stoßimpulsen wenigstens ein beweglich gelagertes Projektil umfasst, welches dazu eingerichtet ist, die Sonde zumindest mittelbar mit Stoßimpulsen zu beaufschlagen. Es kann vorteilhaft eine Energieeffizienz weiter verbesset werden. Insbesondere kann auf eine effiziente Art und Weise ein Impuls hoher Amplitude zur Zertrümmerung von Konkrementen bereitgestellt und verlustarm übertragen werden. Die Stoßeinheit ist insbesondere dazu eingerichtet, das Projektil mechanisch, pneumatisch, hydraulisch und/oder elektromagnetisch zu beschleunigen, wobei vorzugsweise das Projektil seinen durch die Beschleunigung erfahrenen Impuls zumindest teilweise, vorzugsweise zumindest zu einem Großteil und besonders bevorzugt vollständig, als Stoßimpuls auf die Sonde überträgt.

Um vorteilhaft eine Energieeffizienz, insbesondere eine Energieübertragung zwischen Projektil und Sonde, weiter zu verbessern, besteht das Projektil insbesondere wenigstens teilweise, vorzugsweise zumindest zu einem Großteil und besonders bevorzugt vollständig, aus dem amorphen Werkstoff. Ganz besonders bevorzugt ist das Projektil einstückig ausgebildet.

Zudem wird vorgeschlagen, dass die Impulseinheit wenigstens eine Ultraschalleinheit umfasst, welche zur Bereitstellung und Fokussierung von Ultraschallimpulsen wenigstens ein Ultraschallhorn umfasst, welche dazu eingerichtet ist, die Sonde zumindest mittelbar mit Ultraschallimpulsen zu beaufschlagen. Es kann vorteilhaft eine Energieeffizienz weiter verbesset werden. Insbesondere kann auf eine effiziente Art und Weise Impulse hoher Frequenz zur Zertrümmerung von Konkrementen bereitgestellt und verlustarm übertragen werden. Unter einer "Ultraschalleinheit" soll insbesondere eine Einheit verstanden werden, welche dazu ausgebildet ist, Ultraschallimpulse zu erzeugen, bereitzustellen und/oder an ein zu zertrümmerndes Konkrement zu leiten, sowie damit zu beaufschlagen. Unter "Ultraschallimpulse" sollen insbesondere Ultraschallwellen verstanden werden und zwar insbesondere oberhalb eines Hörfrequenzbereichs des Menschen. Im vorliegenden Fall weisen die Ultraschallimpulse insbesondere eine Ultraschallfrequenz von zumindest 10 kHz, vorzugsweise zumindest 15 kHz und besonders bevorzugt zumindest 20 kHz und/oder von höchstens 100 kHz, vorzugsweise von höchstens 80 kHz und besonders bevorzugt von höchstens 56 kHz auf. Ganz besonders bevorzugt beträgt die Ultraschallfrequenz zwischen 22 kHz und 55 kHz. Der Ultraschallgenerator ist insbesondere dabei dazu ausgebildet, Ultraschallimpulse zu erzeugen und vorzugsweise weitere Komponenten der Ultraschalleinheit bereitzustellen. Der Ultraschallgenerator weist insbesondere zumindest einen, vorzugsweise zumindest zwei, besonders bevorzugt zumindest drei und ganz besonders bevorzugt eine Vielzahl von Ultraschallaktoren auf, welcher/welche insbesondere als ein Piezoaktor/Piezoaktoren ausgebildet ist/sind. Insbesondere für den Fall, dass der Ultraschallgenerator zumindest zwei Ultraschallaktoren umfasst, liegen diese zumindest mittelbar aneinander an, um vorteilhaft eine Intensität des erzeugten Ultraschalls zu erhöhen. Der Ultraschallgenerator ist insbesondere mit dem Ultraschallhorn wirkverbunden. Insbesondere um Ultraschallimpulse zu übertragen und zu fokussieren weist das Ultraschallhorn vorzugsweise eine sich vorzugsweise konkav verjüngende Zylinderform auf. Vorzugsweise besteht das Ultraschallhorn zumindest teilweise, vorzugsweise zumindest zu einem Großteil und besonders bevorzugt vollständig aus Metall, wie beispielsweise Eisen, Titan, Stahl oder einer Metalllegierung.

Es wird weiter vorgeschlagen, dass das Ultraschallhorn wenigstens teilweise, insbesondere zumindest zu einem Großteil und besonders bevorzugt vollständig, aus dem amorphen Werkstoff besteht. Es kann vorteilhaft eine Energieeffizienz und/oder eine Bauteileffizienz weiter verbessert werden.

In einem weiteren Aspekt der Erfindung wird ein Lithotripter mit wenigstens einer Lithotripsievorrichtung beansprucht. Es kann vorteilhaft ein Lithotripter mit einer erhöhten Effizienz, insbesondere Energieeffizienz und/oder Bauteileffizienz bereitgestellt werden.

In einem weiteren Aspekt der Erfindung wird ein nicht-therapeuthisches und nichtchirurgisches Verfahren zum Betrieb einer Lithotripsievorrichtung beansprucht. Es kann vorteilhaft ein Lithotripsievorrichtung einer erhöhten Effizienz, insbesondere Energieeffizienz und/oder Bauteileffizienz betrieben werden.

Die erfindungsgemäße Lithotripsievorrichtung, der erfindungsgemäße Lithotripter und/oder das Verfahren zum Betrieb der Lithotripsievorrichtung sollen/soll hierbei nicht auf die oben beschriebene Anwendung und Ausführungsform beschränkt sein. Insbesondere kann die erfindungsgemäße Lithotripsievorrichtung, der erfindungsgemäße Lithotripter und/oder das Verfahren zum Betrieb der Lithotripsievorrichtung zu einer Erfüllung einer hierin beschriebenen Funktionsweise eine von einer hierin genannten Anzahl von einzelnen Elementen, Bauteilen und Einheiten, sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf die Lithotripsievorrichtung beschriebenen Merkmale und Eigenschaften aber auch Verfahrensweisen sinngemäß auf das erfindungsgemäße Verfahrens übertragbar und im Sinne der Erfindung einsetzbar und als mitoffenbart gelten. Gleiches gilt auch in umgekehrter Richtung. Das bedeutet, dass auch in Bezug auf das Verfahren genannte, bauliche, also vorrichtungsgemäße Merkmale im Rahmen der Vorrichtungsansprüche berücksichtigt, beansprucht und ebenfalls zur Offenbarung gezählt werden können.

Falls von einem bestimmten Bauteil mehr als ein Exemplar vorhanden ist, ist nur eines davon in den Figuren und in der Beschreibung mit einem Bezugszeichen versehen. Die Beschreibung dieses Exemplars kann entsprechend auf die anderen Exemplare von dem Bauteil übertragen werden.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung eines Lithotripters mit einer Lithotripsievorrichtung in einer perspektivischen Ansicht,
- Fig. 2: eine schematische Darstellung eines Teils der Lithotripsievorrichtung mit einer Sonde und einer Zertrümmerungseinheit in einer Schnittansicht,
- Fig. 3: eine schematische Darstellung eines Teils der Sonde mit einem Sondenkopf und einem Sondenstab in einer Schnittansicht,
- Fig. 4: eine schematische Darstellung einer atomaren Anordnung eines kristallinen Werkstoffs,
- Fig. 5: eine schematische Darstellung einer atomaren Anordnung eines amorphen Werkstoffs, aus welchem wenigstens teilweise die Zertrümmerungseinheit ausgebildet ist,
- Fig. 6: einen schematischen Ablaufplan eines beispielhaften Verfahrens zur Herstellung der Lithotripsievorrichtung,
- Fig. 7: eine schematische Darstellung einer weiteren Ausgestaltung der Lithotripsievorrichtung mit einer Sonde in einer Schnittansicht,
- Fig. 8: eine schematische Darstellung einer alternativen Ausgestaltung der Lithotripsievorrichtung mit einer Sonde in einer Schnittansicht,
- Fig. 9: eine schematische Darstellung einer zusätzlichen Ausgestaltung der Lithotripsievorrichtung mit einer Sonde in einer Schnittansicht,
- Fig. 10: eine schematische Darstellung einer andersartigen Ausgestaltung der Lithotripsievorrichtung mit einer Sonde in einer Schnittansicht und
- Fig. 11: eine schematische Darstellung einer besonderen Ausgestaltung der Lithotripsievorrichtung mit einer Sonde in einer Schnittansicht.

### Beschreibung der Ausführungsbeispiele

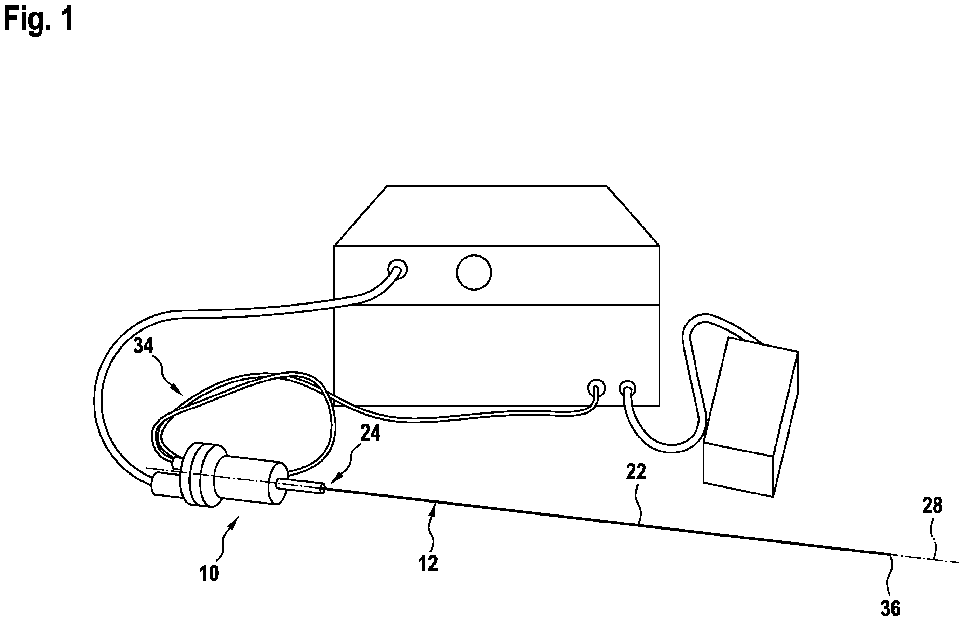

Die Figur 1 zeigt eine schematische Darstellung eines Lithotripters 34 in einer perspektivischen Ansicht. Im vorliegenden Fall ist der Lithotripter 34 als ein intrakorporaler Lithotripter 34 ausgebildet. Der Lithotripter 34 ist dazu eingerichtet zumindest teilweise in Körperöffnungen eingeführt zu werden, um dort angesammelte Konkremente zu zertrümmern und/oder zu entfernen. Der Lithotripter 34 umfasst wenigstens eine Lithotripsievorrichtung. Im vorliegenden Fall bildet die Lithotripsievorrichtung den Lithotripter 34 vollständig aus. Die Lithotriptervorrichtung ist als eine intrakorporale Lithotripsievorrichtung ausgebildet. Alternativ könnte es sich bei dem Lithotripter 34 auch um einen industriellen Lithotripter 34 handeln, welcher beispielsweise zum Zerstören und/oder Dispergieren von Partikeln eingesetzt werden kann.

Die Lithotripsievorrichtung weist eine Zertrümmerungseinheit 10 auf. Die Zertrümmerungseinheit 10 ist zur Beseitigung von Konkrementen eingerichteten. Die Zertrümmerungseinheit 10 weist wenigstens eine Sonde 12 auf. Die Sonde 12 ist dazu eingerichtet Konkremente mit Impulsen zu beaufschlagen. Die Sonde 12 ist im vorliegenden Fall als eine intrakorporale Sonde 12 ausgebildet. Die Sonde 12 ist dazu eingerichtet, um in eine Körperöffnung eingeführt zu werden. Die Sonde 12 ist im vorliegenden Fall stabförmig ausgebildet. Alternativ könnte die Sonde 12 auch röhren und/oder schlauchförmig ausgebildet sein, insbesondere um einen Kanal zum Absaugen und/oder zur Reinigung bereitzustellen.

Die Sonde 12 weist einen Sondenstab 22 auf. Der Sondenstab 22 ist zur wenigstens mittelbaren Beaufschlagung von Konkrementen eingerichtet. Der Sondenstab 22 überträgt Impulse auf eine Sondenspitze 36 der Sonde 12.

Die Sondenspitze 36 ist dazu eingerichtet, Konkremente unmittelbar zu kontaktieren. Die Sondenspitze 36 ist dazu eingerichtet, Impulse auf Konkremente zu übertragen. Die Sondenspitze 36 ist an einem distalen Endabschnitt des Sondenstabs 22 angeordnet. Im vorliegenden Fall sind die Sondenspitze 36 und der Sondenstab 22 einstückig ausgebildet. Alternativ könnten die Sondenspitze 36 und der Sondenstab 22 separat voneinander ausgebildet sein. Ferner wäre denkbar, dass die Sondenspitze 36 beweglich am Sondenstab 22 gelagert ist.

Ferner weist die Sonde 22 zur Aufnahme von Impulsen einen Sondenkopf 24 auf. Der Sondenkopf 24 ist an einem proximalen Endabschnitt des Sondenstabs 22 angeordnet. Der Sondenkopf 24 ist im vorliegenden Fall als eine Hülse ausgebildet.

Im vorliegenden Fall ist der Sondenkopf 24 separat von dem Sondenstab 22 ausgebildet. Der Sondenstab 22 und der Sondenkopf 24 sind wenigstens form- und kraftschlüssig miteinander verbunden. Alternativ könnte der Sondenkopf 24 auch einstückig mit dem Sondenstab 22 verbunden und/oder ausgebildet sein.

Der Sondenkopf 24 umschließt den Sondenstab 22 an dem proximalen Endabschnitt des Sondenstabs 22. Der Sondenkopf 24 weist eine Ausnehmung 38 auf. Die Haupterstreckungsachse 40 der Ausnehmung 38 ist im vorliegenden Fall identisch mit der Haupterstreckungsachse 28 des Sondenstabs 22. Ein größter Innendurchmesser der Ausnehmung 38 ist zumindest im Wesentlichen identisch zu einem kleinsten Außendurchmesser des Sondenstabs 22.

Die Ausnehmung 38 ist eine Teilausnehmung. Die Ausnehmung 38 durchstößt den Sondenstab 22 höchstens teilweise. Die Ausnehmung 38 ist als ein Sackloch ausgebildet. Die Ausnehmung 38 weist eine Haupterstreckungsachse 40 auf. Der Sondenstab 22 ist im Bereich des Endabschnitts in der Ausnehmung 38 angeordnet. Alternativ könnte die Ausnehmung 38 auch eine Vollausnehmung 52 sein und sich vorteilhaft durch die gesamte Erstreckung des Sondenkopfs 24 erstrecken. Ferner könnte sich der Sondenstab 22 wenigstens teilweise über den Sondenkopf 24 heraus erstrecken.

Die Sonde 12 weist einen Eingriffsbereich 26 auf. Im Eingriffsbereich 26 umgibt der Sondenkopf 24 den Sondenstab 22. Der Sondenstab 22 und der Sondenkopf 24 stehen in dem Eingriffsbereich 26 miteinander in Eingriff. Der Eingriffsbereich 26 erstreckt sich über wenigstens 3 mm entlang einer Haupterstreckungsachse 28 des Sondenstabs 22 und/oder des Sondenkopfs 24.

Der Sondenstab 22 weist einen Eingriffsabschnitt 44 auf. Ferner weist der Sondenkopf 24 einen Eingriffsabschnitt 46 auf. Der Eingriffsbereich 26 ist zumindest von dem Eingriffsabschnitt 44 des Sondenstabs 22 und dem Eingriffsabschnitt 46 des Sondenkopfs 24 gebildet. In einem montierten Zustand sind der Sondenkopf 24 und der Sondenstab 22 so zueinander angeordnet, dass der Eingriffsabschnitt 44 des Sondenstabs 22 und der Eingriffsabschnitt 46 des Sondenkopfs 24 zueinander deckungsgleich zueinander angeordnet sind und derart den Eingriffsbereich 26 ausbilden.

Der Eingriffsabschnitt 46 des Sondenkopfs 24 erstreckt sich wenigstens über einen Teil der Ausnehmung 38. Ferner ist denkbar, dass sich der Eingriffsabschnitt 46 über die gesamte Ausnehmung 38 erstreckt. Eine Haupterstreckung des Eingriffsbereichs 26 ist kleiner als eine Haupterstreckung des Sondenkopfs 24. Ferner ist der Eingriffsbereich 26 kleiner als eine Haupterstreckung der Ausnehmung 38.

Im vorliegenden Fall ist der Sondenstab 22 im Eingriffsbereich 26 profiliert. Der Eingriffsabschnitt 44 des Sondenstabs 22 weist wenigstens eine Profilierung 42 auf. Die Profilierung 42 weist wenigstens einen Einstich 48 auf. Bei dem Einstich 48 handelt es sich um einen radialen Einstich 48. Ferner umfasst die Profilierung 42 wenigstens einen weiteren Einstich 48. Der weitere Einstich 48 ist zum Dienst im Wesentlichen identisch zu dem Einstich 48 ausgebildet. Im vorliegenden Fall umfass die Profilierung 42 zwei Einstiche 48. Der weitere Einstich 48 ist entlang der Haupterstreckung des Sondenstabs 22 versetzt zu dem Einstich 48 angeordnet. Ferner könnte der Eingriffsabschnitt 44 des Sondenstabs 22 als Profilierung 42 ein Gewinde, Zähne oder dergleichen aufweisen. Alternativ oder zusätzlich könnte der Sondenkopf 24 im Eingriffsbereich 26 profiliert sein. Dazu könnte der Eingriffsabschnitt 46 des Sondenkopfs 24 eine Profilierung 42 aufweisen. Insbesondere könnte die Profilierung 42 des Eingriffsabschnitts 46 des Sondenkopfs 24 korrespondierend zu der Profilierung 42 des Eingriffsabschnitts 44 des Sondenstabs 22 ausgebildet sein.

Der Sondenkopf 24 weist im Bereich des Eingriffsabschnitts 46 eine verringerte Wandstärke auf. Der Sondenkopf 24 weist wenigstens eine erste Wandstärke auf. Ferner weist der Sondenkopf 24 wenigstens eine zweite Wandstärke auf. Die zweite Wandstärke ist dem Eingriffsabschnitt 44 zugeordnet.

Der Sondenstab 22 und der Sondenkopf 24 sind in einem montierten Zustand durch eine Pressfassung miteinander verbunden. Im vorliegenden Fall ist die Pressfassung eine thermische Pressfassung. Bei der thermischen Pressfassung sind der Sondenstab 22 und der Sondenkopf 24 durch nacheinander Erhitzen und/oder Abkühlen ineinander gefasst und miteinander verbunden. Ferner sind der Sondenstab 22 und der Sondenkopf 24 in einem montierten Zustand axial miteinander im Eingriffsbereich 26 miteinander verpresst. Alternativ oder zusätzlich sind in einem montierten Zustand der Sondenkopfs 24 und des Sondenstabs 22 etwaige Hohlräume und/oder Fugen mit einem Adhäsiv verfüllt.

Ferner weist die Zertrümmerungseinheit 10 wenigstens eine Impulseinheit 14 auf. Die Impulseinheit 14 ist dazu eingerichtet, wenigstens mittelbar Impulse auf die Sonde 12 zu übertragen. Die Impulseinheit 14 weist wenigstens eine Ultraschalleinheit 30 auf. Die Ultraschalleinheit 30 weist wenigstens ein Ultraschallhorn 32 auf. Das Ultraschallhorn 32 dient zur Bereitstellung und Fokussierung von Ultraschallimpulsen. Das Ultraschallhorn 32 ist dazu eingerichtet, die Sonde 12 zumindest mittelbar mit Ultraschallimpulsen zu beaufschlagen. Das Ultraschallhorn 32 weist die Form eines Rotationskörpers auf. Im vorliegenden Fall weist das Ultraschallhorn 32 eine hohlzylinderartige Form auf. Das Ultraschallhorn 32 besteht zumindest teilweise aus Metall, insbesondere Stahl oder Titan.

Die Sonde 12 ist an dem Ultraschallhorn 32 angeordnet. Die Sonde 12 ist mit dem Ultraschallhorn 32 kraft- und/oder formschlüssig verbunden. Im vorliegenden Fall weist das Ultraschallhorn 32 ein Innengewinde auf. Die Sonde 12 weist ein Außengewinde auf. Das Innengewinde der Sonde 12 ist an einer Außenseite des Sondenkopfs 24 angeordnet. Derart ist die Sonde 12 in einem montierten Zustand in das Ultraschallhorn 32 eingedreht.

Die Ultraschalleinheit 30 weist wenigstens einen Ultraschallgenerator 58 auf. Der Ultraschallgenerator 58 ist dazu eingerichtet Ultraschallimpulse bereitzustellen. Der Ultraschallgenerator 58 weist wenigstens einen Ultraschallaktor 54 auf. Im vorliegenden Fall weist der Ultraschallgenerator 58 mehrere Ultraschallaktoren 54 auf. Der Ultraschallgenerator 58 weist drei Ultraschallaktoren 54 auf. Die Ultraschallaktoren 54 sind aneinander anliegend gestapelt angeordnet. Der Übersichtlichkeit halber ist in den Zeichnungen nur ein Ultraschallaktor 54 mit einem Bezugszeichen versehen. Die Ultraschallaktoren 54 sind zumindest im Wesentlichen identisch zueinander ausgebildet. Im Folgenden ist nur ein Ultraschallaktor 54 näher beschrieben. Diese Beschreibung ist auch auf die weiteren Ultraschallaktoren 54 übertragbar. Der Ultraschallaktor 54 weist die Form eines Hohlzylinders auf. Im vorliegenden Fall ist der Ultraschallaktor 54 als ein Piezoaktor 56 ausgebildet. Im vorliegenden Fall sind der Übersichtlichkeit halber etwaige Anschluss- und/oder Kontaktierungsmittel und/oder Isolationen der Ultraschallaktoren 54 nicht dargestellt.

Die Impulseinheit 14 weist wenigstens eine Stoßeinheit 18 auf. Die Stoßeinheit 18 ist zu Bereitstellung von Stoßimpulsen eingerichtet. Die Stoßeinheit 18 umfasst wenigstens ein beweglich gelagertes Projektil 20. Das Projektil 20 ist dazu eingerichtet, die Sonde 12 zumindest mittelbar mit Stoßimpulsen zu beaufschlagen. Die Stoßeinheit 18 ist dazu eingerichtet das Projektil 20 zu beschleunigen. Im vorliegenden Fall beschleunigt die Stoßeinheit 18 das Projektil 20 pneumatisch. Dazu weist die Stoßeinheit 18 einen Projektilkanal auf. In dem Projektilkanal ist das Projektil 20 angeordnet. Das Projektil 20 liegt fluiddicht an einer Wandung des Projektilkanals an. Ferner weist die Stoßeinheit 18 einen Fluidanschluss auf. Der Fluidanschluss ist fluidtechnisch mit dem Projektilkanal verbunden. Der Fluidanschluss ist mit einer Pneumatikeinheit der Lithotripsievorrichtung verbunden, welches der Stoßeinheit 18 pneumatische Energie in Form von Pressluft zur Verfügung stellt. Derart kann das Projektil 20 durch Pressluft innerhalb des Projektilkanals beschleunigt werden. Trifft das Projektil 20 auf die Sonde 12 überträgt das Projektil 20 seinen durch die Beschleunigung erfahrenen Impuls als Stoßimpuls zumindest zu einem Großteil auf die Sonde 12. Alternativ oder zusätzlich könnte die Stoßeinheit 18 zur mechanischen, hydraulischen und/oder elektromagnetischen Beschleunigung des Projektils 20 eingerichtet sein.

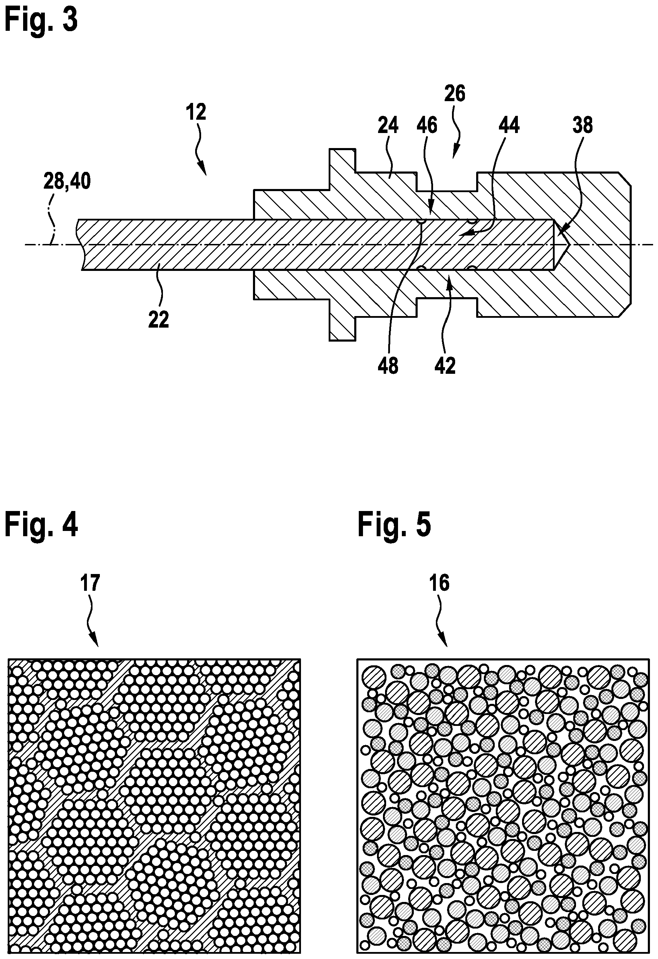

Die Zertrümmerungseinheit 10 besteht wenigstens teilweise aus einem amorphen Werkstoff 16. In Fig. 5 ist eine schematische Darstellung einer strukturellen Anordnung von Atomen in dem amorphen Werkstoff 16 gezeigt. Im Vergleich dazu zeigt Fig. 4 eine schematische Darstellung einer strukturellen Anordnung von Atomen in einem von dem amorphen Werkstoff 16 verschiedenen kristallinen Werkstoff 17.

Der amorphe Werkstoff 16 weist eine Elastizitätsgrenze von wenigstens 1,5% auf. Ferner weist der amorphe Werkstoff 16 eine Festigkeit von wenigstens 500 MPa auf. Der amorphe Werkstoff 16 ist metallisches Glas ist. Der amorphe Werkstoff 16 weist wenigstens ein Metall auf. Im vorliegenden Fall weist der amorphe Werkstoff 16 als Metalle Kupfer und Aluminium auf. Ferner weist der amorphe Werkstoff 16 wenigstens ein Übergangsmetall auf. Im vorliegenden Fall weist der vorliegende amorphe Werkstoff 16 als Übergangsmetalle wenigstens Zirconium, Titan und Nickel auf. Der amorphe Werkstoff 16 umfasst also ein Gemisch aus Metallen und Übergangsmetallen. Im vorliegenden Fall ist der amorphe Werkstoff 16 frei von Erdalkalimetallen. Alternativ könnte der amorphe Werkstoff 16 auch Erdalkalimetalle umfassen.

Der amorphe Werkstoff 16 ist im vorliegenden Fall Zirconium basiert. Zirconium bildet einen größten Anteil des amorphen Werkstoffs 16 aus. Der amorphe Werkstoff 16 weist wenigstens 40 % Zirconium auf. Im vorliegenden Fall weist der amorphe Werkstoff 16 sogar wenigstens 50 % Zirconium auf. Alternativ könnte der amorphe Werkstoff 16 auch Kupfer und/oder Titan basiert sein. Im vorliegenden Fall ist als amorpher Werkstoff 16 Zr_{52,5}Cu_{17,9}Ni_{14,6}Al₁₀Ti₅ (at%) gewählt. Der amorphe Werkstoff 16 ist bekannt unter dem Markennamen Vitreloy^{™} 105. Alternativ könnte auch ein anderer amorpher Werkstoff 16 genutzt werden.

Die Sonde 12 besteht wenigstens teilweise aus dem amorphen Werkstoff 16. Der Sondenkopf 24 besteht zumindest teilweise aus dem amorphen Werkstoff 16. Im vorliegenden Fall besteht der Sondenkopf 24 sogar vollständig aus dem amorphen Werkstoff 16. Der Sondenstab 22 hingegen ist im vorliegenden Fall frei von dem amorphen Werkstoff 16. Alternativ oder zusätzlich könnte auch der Sondenstab 22 zumindest teilweise aus dem amorphen Werkstoff 16 bestehen.

Die Impulseinheit 14 besteht wenigstens teilweise aus dem amorphen Werkstoff 16. Im vorliegenden Fall besteht die Stoßeinheit 18 der Impulseinheit 14 wenigstens teilweise aus dem amorphen Werkstoff 16. Das Projektil 20 der Stoßeinheit 18 besteht wenigstens teilweise aus dem amorphen Werkstoff 16. Im vorliegenden Fall besteht das Projektil 20 sogar vollständig aus dem amorphen Werkstoff 16. Alternativ oder zusätzlich könnte die Ultraschalleinheit 30 wenigstens teilweise aus dem amorphen Werkstoff 16 bestehen. Beispielsweise kann das Ultraschallhorn 32 der Ultraschalleinheit 30 wenigstens teilweise aus dem amorphen Werkstoff 16 bestehen.

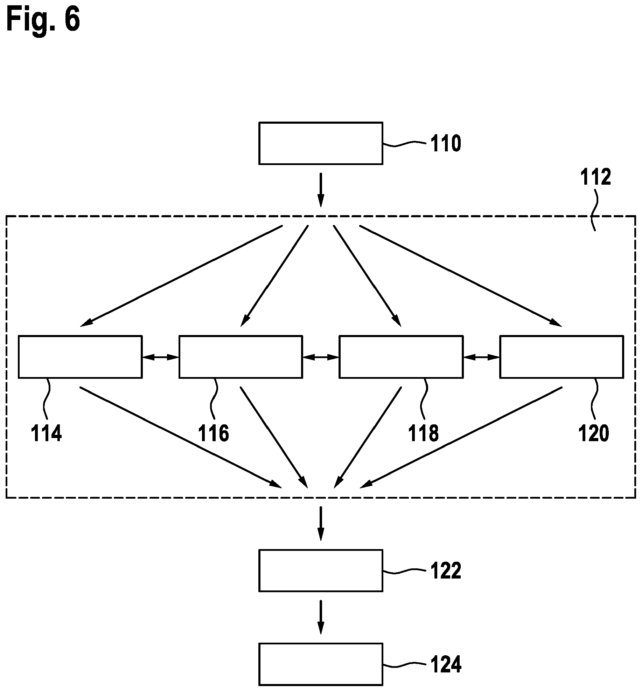

Fig. 6 zeigt einen schematischen Ablaufplan eines beispielhaften Verfahrens zur Herstellung der Lithotripsievorrichtung. In dem Verfahren wird die Zertrümmerungseinheit 10 wenigstens teilweise aus dem amorphen Werkstoff 16 ausgebildet.

Das Verfahren umfasst wenigsten einen Verfahrensschritt 110. In dem Verfahrensschritt 110 werden die aus dem amorphen Werkstoff 16 bestehenden Komponenten der Zertrümmerungseinheit 10 hergestellt. Als Komponenten werden zumindest die Sonde 12 und/oder die Impulseinheit 14 aus dem amorphen Werkstoff 16 hergestellt. Bei der vorliegenden Sonde 12 wird wenigstens der Sondenkopf 24 zumindest teilweise aus dem amorphen Werkstoff 16 hergestellt. Bei der vorliegenden Impulseinheit 14 werden ferner die Stoßeinheit 18 und/oder die Ultraschalleinheit 30 zumindest teilweise aus dem amorpher Werkstoff 16 hergestellt. Aus dem amorphen Werkstoff 16 wird das Projektil 20 der Stoßeinheit 18 hergestellt. Ferner könnte das Ultraschallhorn 32 wenigstens teilweise aus dem amorphen Werkstoff 16 hergestellt werden. Im vorliegenden Fall werden die Komponenten durch Urformen, insbesondere Spritzgießen hergestellt. Alternativ oder zusätzlich könnten die Komponenten auch durch Laserschmelzen, Lasersintern oder dergleichen hergestellt werden.

Das Verfahren umfasst wenigstens einen weiteren Verfahrensschritt 112. In dem weiteren Verfahrensschritt 112 werden aus dem amorphen Material hergestellte Komponenten mit weiteren Bauteilen der Lithotripsievorrichtung verbunden. Beispielsweise wird der Sondenkopf 24 wird mit dem Sondenstab 22 verbunden.

Der Verfahrensschritt 112 umfasst wenigstens einen Teilverfahrensschritt 114. In dem Teilverfahrensschritt 114 wird der Sondenkopf 24 form- und/oder kraftschlüssig mit dem Sondenstab 22 verbunden. Diese werden mittels einer Pressfassung miteinander verbunden. Im vorliegenden Fall ist die Pressfassung eine thermische Pressfassung. Der Sondenkopf 24 wird im Verhältnis zu der Temperatur des Sondenstabs 22 erhitzt. Durch Erhitzen des Sondenkopfs 24 wird die Ausnehmung 38 des Sondenkopfs 24 geweitet, sodass der Sondenstab 22 in diesen eingeführt werden kann. Der Sondenstab 22 wird in den Sondenkopf 24 soweit eingeführt, dass der Eingriffsabschnitt 44 des Sondenstabs 22 deckungsgleich mit dem Eingriffsabschnitt 46 des Sondenkopfs 24 angeordnet ist. Ist die Ausnehmung 38 als ein Sackloch ausgebildet wird der Sondenstab 22 ferner soweit in den Sondenkopf 24 eingeführt, bis dieser an eine Endfläche der Ausnehmung 38 anstößt. Der Sondenkopf 24 wird abgekühlt, wodurch sich dessen Ausnehmung 38 verengt. Der Sondenstab 22 wird mit dem Sondenkopf 24 radial verpresst. Dadurch werden der Sondenkopf 24 und der Sondenstab 22 ineinander verkeilt. Dabei greift die Profilierung 42 des Endabschnitts des Sondenstabs 22 in den Endabschnitt 46 des Sondenkopfs 24 ein. Im Endabschnittsbereich 26 greifen somit der Sondenkopf 24 und der Sondenstab 22 ineinander ein.

Der Verfahrensschritt 112 kann einen weiteren Teilverfahrensschritt 116 umfassen. Dieser kann zusätzlich oder alternativ zu dem Teilverfahrensschritt 114 durchgeführt werden. Der in dem Sondenkopf 24 eingeführte Sondenstab 22 wird wenigstens im Eingriffsbereich 26 radial mit einer externen Kraft beaufschlagt, wodurch dieser im Eingriffsbereich 26 mit dem Sondenstab 22 vercrimpt wird.

Der Verfahrensschritt 112 kann einen weiteren Teilverfahrensschritt 118 umfassen. Dieser kann zusätzlich oder alternativ zu dem Teilverfahrensschritt 114 und/oder zu dem Teilverfahrensschritt 116 durchgeführt werden. In diesem Teilverfahrensschritt 118 werden der ineinander eingeführte Sondenstab 22 und Sondenkopf 24 stoffschlüssig miteinander verbunden. Dazu wird ein Adhäsiv genutzt, mittels welchem Fugen und/oder Ausnehmungen der Sonde 12, insbesondere zwischen dem Sondenstab 22 und dem Sondenkopf 24 gefüllt werden.

Der Verfahrensschritt 112 kann einen weiteren Teilverfahrensschritt 120 umfassen. Dieser kann zusätzlich oder alternativ zu dem Teilverfahrensschritt 114, zu dem Teilverfahrensschritt 116 und/oder zu dem Teilverfahrensschritt 118 durchgeführt werden. Ferner kann der Teilverfahrensschritt 120 gleichzeitig mit dem Teilverfahrensschritt 114, mit dem Teilverfahrensschritt 116 und/oder mit dem Teilverfahrensschritt 118 ausgeführt werden. In dem Teilverfahrensschritt 120 wird der Sondenkopf 24 gleichzeitig Hergestellt und mit dem Sondenstab 22 form- und/oder kraftschlüssig mit dem Sondenkopf 24 verbunden. Der Sondenstab 22 wird mit dem Sondenkopf 24 umspritzt.

Das Verfahren umfasst wenigstens einen weiteren Verfahrensschritt 122. In dem weiteren Verfahrensschritt 122 werden die zuvor aus dem amorphen Werkstoff 16 hergestellt und/oder verbundenen Komponenten mit weiteren Komponenten der Lithotripsievorrichtung verbunden. Die Sonde 12 wird mit dem Ultraschallhorn 32 verbunden. Ferner wir das Projektil 20 in den Projektilkanal eingelegt.

Das Verfahren umfasst wenigstens einen weiteren Verfahrensschritt 124. In dem weiteren Verfahrensschritt 124 wird die Impulseinheit 14 in einem Gehäuse angeordnet.

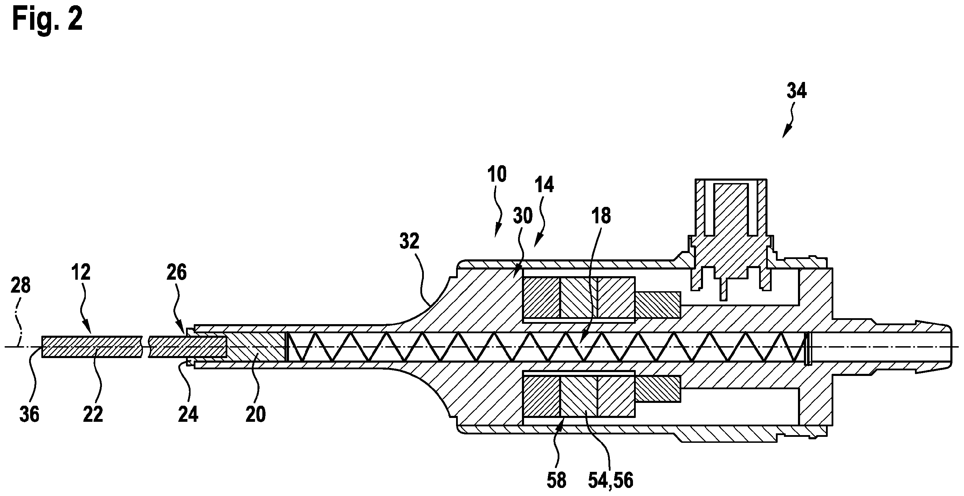

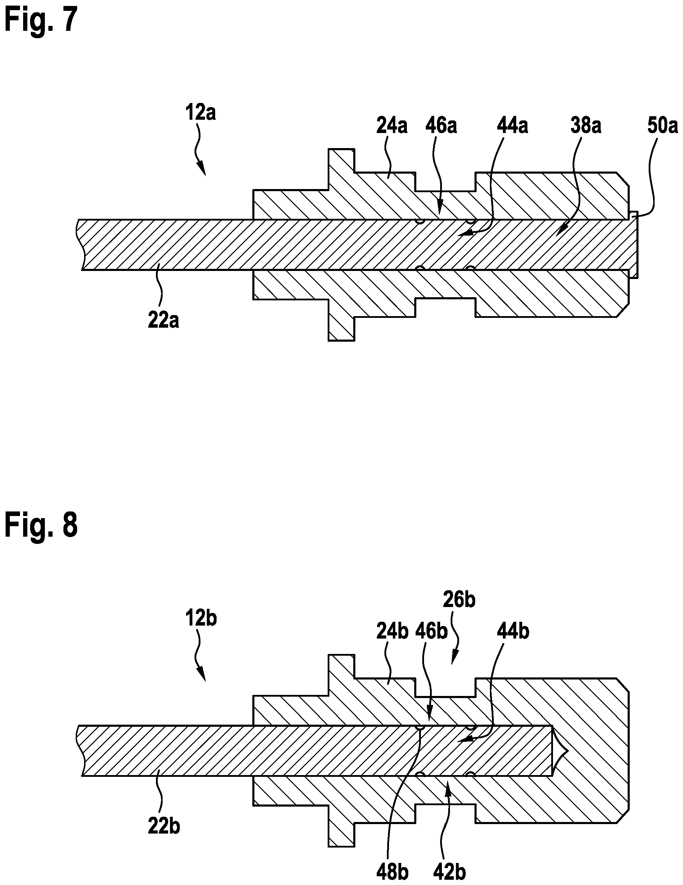

In den Figuren 7 bis 11 sind Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispielen, insbesondere der Figuren 1 bis 6 verwiesen wird. Zur Unterscheidung der weiteren Ausführungsbeispiele der Figuren 7 bis 11 ist den Bezugszeichen die Buchstaben a, b, c, d und e nachgestellt, wobei sich gleiche Bezugszeichen auf die entsprechenden Bauteile des vorhergehenden Ausführungsbeispiels beziehen.

Fig.7 zeigt eine schematische Darstellung einer weiteren Ausgestaltung der Lithotripsievorrichtung mit einer Sonde 12a in einer Schnittansicht. Das vorliegende Ausführungsbeispiel unterscheidet sich im Wesentlichen von dem Vorhergehenden durch eine Ausgestaltung der Sonde 12a.

Im vorliegenden Fall weist ein Sondenkopf 24a der Sonde 12a eine Ausnehmung 38a auf, welche als eine Vollausnehmung 52a ausgebildet ist. Der Sondenstab 22a weist eine Verbreiterung 50a auf. Die Verbreiterung 50a ist an einem proximalen Endabschnitt des Sondenstabs 22a angeordnet. Die Verbreiterung 50a bildet einen Anschlag aus. Mittels der Verbreiterung 50a liegt der Sondenstab 22a stirnseitig an einer proximalen Seite des Sondenkopfs 24a an. Durch das Anliegen der Verbreiterung 50a an der proximalen Stirnseite des Sondenkopfs 24a kann zusätzlich ein ungewolltes Auslösen der Verbindung zwischen Sondenkopf 24a und Sondenstab 22a vermieden werden. Vorteilhaft kann vermieden werden, dass sich im Betrieb der Sondenstab 22a in Distalrichtung löst, wobei er wohlmöglich in einem Patienten stecken bleiben könnte.

Alternativ oder zusätzlich zu dem Verfahren des vorhergehenden Ausführungsbeispiels wird bei der Herstellung der vorliegenden Lithotripsievorrichtung der Sondenstab 22a proximalseitig in den Sondenkopf 24a eingeführt. Stößt die Verbreiterung 50a an die proximale Stirnseite des Sondenstabs 22a an, so sind Eingriffsabschnitte 44a, 46a des Sondenstabs 22a und des Sondenkopf 24a miteinander deckungsgleich angeordnet. Die Verbreiterung 50a des Sondenstabs 22a könnte mittels Urformen hergestellt werden. Vorzugsweise wird die Verbreiterung 50a jedoch durch Umformen, wie beispielsweise dengeln erzielt.

Fig.8 zeigt eine schematische Darstellung einer alternativen Ausgestaltung der Lithotripsievorrichtung mit einer Sonde 12b in einer Schnittansicht. Das vorliegende Ausführungsbeispiel unterscheidet sich im Wesentlichen von dem vorhergehenden durch eine Ausgestaltung der Sonde 12b.

Die Sonde 12b weist einen Sondenkopf 24b auf. Ferner weist die Sonde 12b einen Sondenstab 22b auf. Im vorliegenden Fall ist der Sondenstab 22b und der Sondenkopf 24b miteinander stoffschlüssig verbunden. Die Sonde 12b weist einen Eingriffsbereich 26b auf. Der Sondenstab 22b weist einen Eingriffsabschnitt 44b auf. Der Eingriffsabschnitt 44b bildet den Eingriffsbereich 26b teilweise aus. In dem Eingriffsabschnitt 44b weist der Sondenstab 22b eine Profilierung 42b auf. Die Profilierung 42b ist im vorliegenden Fall in von wenigstens zwei Einstichen 48b ausgebildet. Ferner weist der Sondenkopf 24b einen Eingriffsabschnitt 46b auf. Der Eingriffsabschnitt 46b bildet zumindest teilweise den Eingriffsbereich 26b aus.

Alternativ oder zusätzlich zu dem Verfahren des vorhergehenden Ausführungsbeispiels wird bei der Herstellung der vorliegenden Lithotripsievorrichtung der Sondenstab 22b mit dem Sondenkopf 24b umgossen. Ferner könnte auch der Sondenstab 22b in den Sondenkopf 24b hineingegossen werden. Das Material des Sondenkopfs 24b umgibt die Profilierung 42b des Eingriffsabschnitts 46b des Sondenkopfs 24b. Das Material des Sondenkopfs 24b verfüllt die Einstiche 48b.

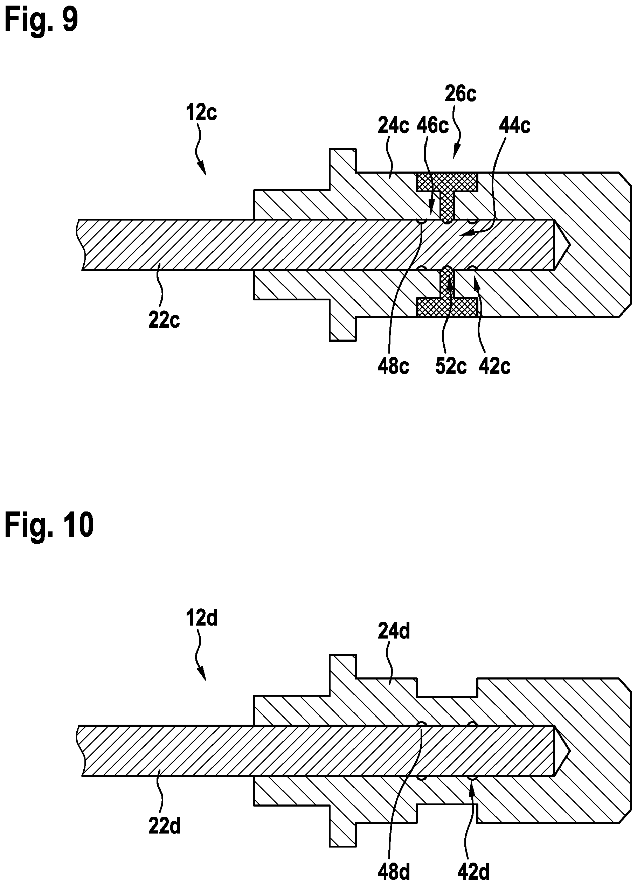

Fig. 9 zeigt eine schematische Darstellung einer zusätzlichen Ausgestaltung der Lithotripsievorrichtung mit einer Sonde 12c in einer Schnittansicht. Das vorliegende Ausführungsbeispiel unterscheidet sich im Wesentlichen von dem vorhergehenden durch eine Ausgestaltung der Sonde 12c.

Die Sonde 12c weist einen Sondenkopf 24c auf. Ferner weist die Sonde 12c einen Sondenstab 22c auf. In einem Eingriffsbereich 26 der Sonde 12c sind der Sondenkopf 24 und der Sondenstab 22c miteinander verbunden. Der Sondenstab 22c weist einen Eingriffsabschnitt 46c auf. Der Sondenstab 22c weist einen Eingriffsabschnitt 44c auf. Der Eingriffsabschnitt 44c bildet den Eingriffsbereich 26c teilweise aus. In dem Eingriffsabschnitt 44c weist der Sondenstab 22c eine Profilierung 42c auf. Die Profilierung 42c ist im vorliegenden Fall in von wenigstens zwei Einstichen 48c ausgebildet. Ferner weist die Profilierung 42c einen zusätzlichen Einstich 48c auf. Der zusätzliche Einstich 48c ist mittig zwischen den beiden Einstichen 48c angeordnet.

Der Sondenkopf 24c weist senkrecht zu seiner Haupterstreckungsachse 28c eine Vollausnehmung 52c im Bereich des Eingriffsabschnitts 46c auf. Die Vollausnehmung 52c ist zu einer Verfüllung mit einem Adhäsiv vorgesehen. Sind ein Eingriffsabschnitt 26c der Sonde 12c und ein Eingriffsabschnitt 26c des Sondenkopfs 24c miteinander deckungsgleich angeordnet, liegt die Ausnehmung 38c deckungsgleich zu dem zusätzlichen Einstich 48c der Profilierung 42c. Die Vollausnehmung 52c und der zusätzliche Einstich 48c sind bei einer adhäsiven Verbindung des Sondenkopfs 24c mit dem Sondenstab 22c mit dem Adhäsiv verfüllt.

Alternativ oder zusätzlich zu dem Verfahren der vorhergehenden Ausführungsbeispiele wird bei der Herstellung der vorliegenden Lithotripsievorrichtung der Sondenstab 22c mit dem Sondenkopf 24c adhäsiv verbunden. Ein Adhäsiv wird in die Ausnehmung 38c eingefüllt. Dabei fließt das Adhäsiv in den weiteren Einstich 48c der Profilierung 42c.

Fig.10 eine schematische Darstellung einer andersartigen Ausgestaltung der Lithotripsievorrichtung mit einer Sonde 12d in einer Schnittansicht. Das vorliegende Ausführungsbeispiel unterscheidet sich im Wesentlichen von dem vorhergehenden durch eine Ausgestaltung der Sonde 12d.

Die Sonde 12d weist einen Sondenkopf 24d auf. Ferner weist die Sonde 12d einen Sondenstab 22d auf. Im vorliegenden Fall weist der Sondenkopf 24d eine Profilierung 42d auf. Die Profilierung 42d umfasst zwei versetzt zueinander angeordnete Einstiche 48d.

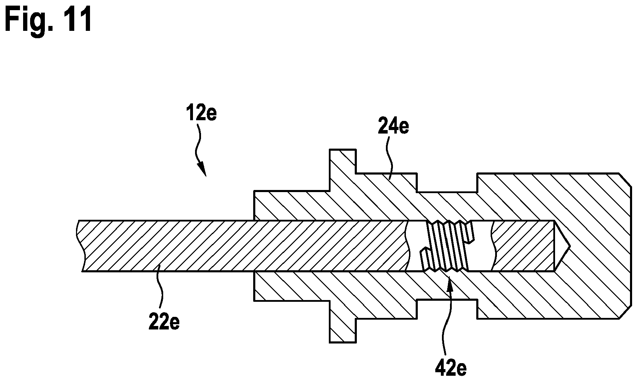

Fig.11 zeigt eine schematische Darstellung einer besonderen Ausgestaltung der Lithotripsievorrichtung mit einer Sonde 12e in einer Schnittansicht. Das vorliegende Ausführungsbeispiel unterscheidet sich im Wesentlichen von dem Vorhergehenden durch eine Ausgestaltung der Sonde 12e.

Die Sonde 12e weist einen Sondenkopf 24e auf. Ferner weist die Sonde 12e beinen Sondenstab 22e auf. Im vorliegenden Fall weist der Sondenstab 22e eine Profilierung 42e auf. Die Profilierung 42e ist im vorliegenden Fall als ein Gewinde ausgebildet.

| | | | |
|---|---|---|---|
| 10 | Zertrümmerungseinheit | 50 | Verbreiterung |
| 12 | Sonde | 52 | Vollausnehmung |
| 14 | Impulseinheit | 54 | Ultraschallaktor |
| 16 | amorpher Werkstoff | 56 | Piezoaktor |
| 17 | kristalliner Werkstoff | 58 | Ultraschallgenerator |
| 18 | Stoßeinheit | 110 | Verfahrensschritt |
| 20 | Projektil | 112 | Verfahrensschritt |
| 22 | Sondenstab | 114 | Teilverfahrensschritt |
| 24 | Sondenkopf | 116 | Teilverfahrensschritt |
| 26 | Eingriffsbereich | 118 | Teilverfahrensschritt |
| 28 | Haupterstreckungsachse | 120 | Teilverfahrensschritt |
| 30 | Ultraschalleinheit | 122 | Verfahrensschritt |
| 32 | Ultraschallhorn | 124 | Verfahrensschritt |
| 34 | Lithotripter | | |
| 36 | Sondenspitze | | |
| 38 | Ausnehmung | | |
| 40 | Haupterstreckungsachse | | |
| 42 | Profilierung | | |
| 44 | Eingriffsabschnitt | | |
| 46 | Eingriffsabschnitt | | |
| 48 | Einstich | | |

## Patentansprüche

1. Lithotripsievorrichtung, insbesondere intrakorporale Lithotripsievorrichtung, mit wenigstens einer Zertrümmerungseinheit (10), welche zur Beseitigung von Konkrementen eingerichtet ist und welche wenigstens eine zur Beaufschlagung von Konkrementen eingerichtete Sonde (12a-e), sowie wenigstens eine zumindest mittelbar Impulse auf die Sonde (12a-e) übertragende Impulseinheit (14) umfasst, welche zur wenigstens mittelbaren Beaufschlagung von Konkrementen einen Sondenstab (22a-e), sowie zur Aufnahme von Impulsen einen Sondenkopf (24a-e) aufweist, **dadurch gekennzeichnet, dass** die Zertrümmerungseinheit (10) wenigstens teilweise aus einem amorphen Werkstoff (16) besteht, welcher eine Elastizitätsgrenze von wenigstens 1,5% und/oder eine Festigkeit von wenigstens 500 MPa aufweist, wobei der amorphe Werkstoff (16) metallisches Glas ist.

2. Lithotripsievorrichtung nach Anspruch 1, **dadurch gekennzeichnet, dass** der amorphe Werkstoff (16) wenigstens ein Metall, wenigstens ein Übergangsmetall, wenigstens ein Erdalkalimetall und/oder ein Gemisch dieser umfasst.

3. Lithotripsievorrichtung nach Anspruch 1 oder 2, **dadurch gekennzeichnet, dass** der amorphe Werkstoff (16) Zirconium basiert ist.

4. Lithotripsievorrichtung nach einem der vorhergehenden Ansprüche, **dadurch gekennzeichnet, dass** die Sonde (12a-e) wenigstens teilweise aus dem amorphen Werkstoff (16) besteht.

5. Lithotripsievorrichtung nach einem der vorhergehenden Ansprüche, **dadurch gekennzeichnet, dass** der Sondenkopf (24a-e) zumindest teilweise aus dem amorphen Werkstoff (16) besteht.

6. Lithotripsievorrichtung nach einem der vorhergehenden Ansprüche, **dadurch gekennzeichnet, dass** der Sondenstab (22a-e) frei von dem amorphen Werkstoff (16) ist.

7. Lithotripsievorrichtung nach Anspruch 1 und insbesondere nach einem der vorhergehenden Ansprüche, **dadurch gekennzeichnet, dass** der Sondenstab (22a-e) und der Sondenkopf (24a-e) wenigstens form- und/oder kraftschlüssig, insbesondere reibschlüssig, miteinander verbunden sind.

8. Lithotripsievorrichtung nach Anspruch 7, **dadurch gekennzeichnet, dass** die Sonde (12a-e) einen Eingriffsbereich (26b; 26c) aufweist, in welchem der Sondenstab (22a-e) und der Sondenkopf (24a-e) miteinander in Eingriff stehen.

9. Lithotripsievorrichtung nach Anspruch 8, **dadurch gekennzeichnet, dass** der Eingriffsbereich (26b; 26c) sich über wenigstens 3 mm entlang einer Haupterstreckungsachse (28c/40c) des Sondenstabs (22a-e) und der Sondenkopfs (24a-e) erstreckt.

10. Lithotripsievorrichtung nach 7 oder 8, **dadurch gekennzeichnet, dass** im Eingriffsbereich (26b: 26c) der Sondenkopf (24a-e) den Sondenstab (22a-e) umgibt.

11. Lithotripsievorrichtung nach einem der Ansprüche 7 bis 9, **dadurch gekennzeichnet, dass** im Eingriffsbereich (26b,c) der Sondenstab (22a-e) und/oder der Sondenkopf (24a-e) profiliert ist/sind.

12. Lithotripsievorrichtung nach einem der Ansprüche 7 bis 9, **dadurch gekennzeichnet, dass** der Sondenstab (22a-e) und der Sondenkopf (24a-e) miteinander verpresst, insbesondere vercrimpt, sind.

13. Lithotripsievorrichtung nach einem der vorhergehenden Ansprüche, **dadurch gekennzeichnet, dass** der Sondenstab (22a-e) und der Sondenkopf (24a-e) miteinander stoffschlüssig verbunden sind.

14. Lithotripsievorrichtung nach Anspruch 13, **dadurch gekennzeichnet, dass** der Sondenstab (22a-e) und der Sondenkopf (24a-e) miteinander umspritzt sind.

15. Lithotripsievorrichtung nach einem der vorhergehenden Ansprüche, **dadurch gekennzeichnet, dass** die Impulseinheit (14) wenigstens eine Stoßeinheit (18) umfasst, welche zur Bereitstellung von Stoßimpulsen wenigstens ein beweglich gelagertes Projektil (20) umfasst, welches dazu eingerichtet ist, die Sonde (12a-e) zumindest mittelbar mit Stoßimpulsen zu beaufschlagen.

16. Lithotripsievorrichtung nach Anspruch 15, **dadurch gekennzeichnet, dass** das Projektil (20) wenigstens teilweise aus dem amorphen Werkstoff (16) besteht.

17. Lithotripsievorrichtung nach einem der vorhergehenden Ansprüche, **dadurch gekennzeichnet, dass** die Impulseinheit (14) wenigstens eine Ultraschalleinheit (30) umfasst, welche zur Bereitstellung und Fokussierung von Ultraschallimpulsen wenigstens ein Ultraschallhorn (32) umfasst, welches dazu eingerichtet ist, die Sonde (12a-e) zumindest mittelbar mit Ultraschallimpulsen zu beaufschlagen.

18. Lithotripsievorrichtung nach Anspruch 17, **dadurch gekennzeichnet, dass** das Ultraschallhorn (32) wenigstens teilweise aus dem amorphen Werkstoff (16) besteht.

19. Lithotripter (34) mit wenigstens einer Lithotripsievorrichtung nach einem der vorhergehenden Ansprüche.

20. Verfahren zum nicht-therapeutischen und nicht-chirurgischen Betrieb einer Lithotripsievorrichtung nach einem der vorhergehenden Ansprüche.

## Claims

1. A lithotripsy device, in particular intracorporeal lithotripsy device, having at least one disintegration unit (10) which is configured for removing concrements and which comprises at least one probe (12a-e) configured for acting on concrements, and at least one pulse unit (14), which at least indirectly transmits pulses to the probe (12a-e) and which has a probe rod (22a-e) for at least indirectly acting on concrements and a probe head (24a-e) for receiving pulses, **characterized in that** the disintegration unit (10) at least partially consists of an amorphous material (16) which has an elastic limit of at least 1.5% and/or a strength of at least 500 MPa, wherein the amorphous material (16) is metallic glass.

2. The lithotripsy device according to claim 1, **characterized in that** the amorphous material (16) comprises at least one metal, at least one transition metal, at least one alkaline earth metal, and/or a mixture thereof.

3. The lithotripsy device according to claim 1 or 2, **characterized in that** the amorphous material (16) is based on zirconium.

4. The lithotripsy device according to one of the preceding claims, **characterized in that** the probe (12a-e) at least partially consists of the amorphous material (16).

5. The lithotripsy device according to one of the preceding claims, **characterized in that** the probe head (24a-e) at least partially consists of the amorphous material (16).

6. The lithotripsy device according to one of the preceding claims, **characterized in that** the probe rod (22a-e) is free of the amorphous material (16).

7. The lithotripsy device according to claim 1 and in particular according to one of the preceding claims, **characterized in that** the probe rod (22a-e) and the probe head (24a-e) are connected to one another at least in a positive and/or non-positive manner, in particular in a frictional manner.

8. The lithotripsy device according to claim 7, **characterized in that** the probe (12a-e) has an engagement region (26b; 26c) in which the probe rod (22a-e) and the probe head (24a-e) are engaged with one another.

9. The lithotripsy device according to claim 8, **characterized in that** the engagement region (26b; 26c) extends for at least 3 mm along a main axis of extension (28c/40c) of the probe rod (22a-e) and the probe head (24a-e).

10. The lithotripsy device according to 7 or 8, **characterized in that** the probe head (24a-e) surrounds the probe rod (22a-e) in the engagement region (26b: 26c).

11. The lithotripsy device according to one of claims 7 to 9, **characterized in that** the probe rod (22a-e) and/or the probe head (24a-e) is/are profiled in the engagement region (26b,c).

12. The lithotripsy device according to one of claims 7 to 9, **characterized in that** the probe rod (22a-e) and the probe head (24a-e) are pressed, in particular crimped, together.

13. The lithotripsy device according to one of the preceding claims, **characterized in that** the probe rod (22a-e) and the probe head (24a-e) are connected to one another in a materially-bonded manner.

14. The lithotripsy device according to claim 13, **characterized in that** the probe rod (22a-e) and the probe head (24a-e) are overmolded with one another.

15. The lithotripsy device according to one of the preceding claims, **characterized in that** the pulse unit (14) comprises at least one impact unit (18), which comprises at least one movably mounted projectile (20) for providing impact pulses, said projectile being configured for at least indirectly applying impact pulses to the probe (12a-e).

16. The lithotripsy device according to claim 15, **characterized in that** the projectile (20) at least partially consists of the amorphous material (16).

17. The lithotripsy device according to one of the preceding claims, **characterized in that** the pulse unit (14) comprises at least one ultrasound unit (30), which comprises at least one ultrasonic horn (32) for providing and focusing ultrasonic pulses, said ultrasonic horn being configured for at least indirectly applying ultrasonic pulses to the probe (12a-e).

18. The lithotripsy device according to claim 17, **characterized in that** the ultrasonic horn (32) at least partially consists of the amorphous material (16).

19. A lithotripter (34) having at least one lithotripsy device according to one of the preceding claims.

20. A method for non-therapeutic and non-surgical operation of a lithotripsy device according to one of the preceding claims.

## Revendications

1. Dispositif de lithotripsie, notamment dispositif de lithotripsie intracorporelle, comprenant au moins une unité de fragmentation (10), qui est conçue pour l'élimination de concrétions et qui comprend au moins une sonde (12a-e) conçue pour l'impact sur des concrétions, ainsi qu'au moins une unité d'impulsion (14) transmettant au moins indirectement des impulsions à la sonde (12a-e), qui comprend une tige de sonde (22a-e) pour l'impact au moins indirect sur des concrétions, ainsi qu'une tête de sonde (24a-e) pour recevoir des impulsions, **caractérisé en ce que** l'unité de fragmentation (10) est au moins partiellement constituée d'un matériau amorphe (16), qui présente une limite d'élasticité d'au moins 1,5 % et/ou une résistance d'au moins 500 MPa, dans lequel le matériau amorphe (16) est du verre métallique.

2. Dispositif de lithotripsie selon la revendication 1, **caractérisé en ce que** le matériau amorphe (16) comprend au moins un métal, au moins un métal de transition, au moins un métal alcalino-terreux et/ou un mélange de ceux-ci.

3. Dispositif de lithotripsie selon la revendication 1 ou 2, **caractérisé en ce que** le matériau amorphe (16) est à base de zirconium.

4. Dispositif de lithotripsie selon l'une quelconque des revendications précédentes, **caractérisé en ce que** la sonde (12a-e) est au moins partiellement constituée du matériau amorphe (16).

5. Dispositif de lithotripsie selon l'une quelconque des revendications précédentes, **caractérisé en ce que** la tête de sonde (24a-e) est au moins partiellement constituée du matériau amorphe (16).

6. Dispositif de lithotripsie selon l'une quelconque des revendications précédentes, **caractérisé en ce que** la tige de sonde (22a-e) est exempte du matériau amorphe (16).

7. Dispositif de lithotripsie selon la revendication 1 et notamment selon l'une quelconque des revendications précédentes, **caractérisé en ce que** la tige de sonde (22a-e) et la tête de sonde (24a-e) sont reliées au moins par complémentarité de forme et/ou de force, notamment par friction, l'une à l'autre.

8. Dispositif de lithotripsie selon la revendication 7, **caractérisé en ce que** la sonde (12a-e) comprend une partie de mise en prise (26b ; 26c), dans laquelle la tige de sonde (22a-e) et la tête de sonde (24a-e) sont en prise l'une avec l'autre.

9. Dispositif de lithotripsie selon la revendication 8, **caractérisé en ce que** la partie de mise en prise (26b ; 26c) s'étend sur au moins 3 mm le long d'un axe d'extension principal (28c/40c) de la tige de sonde (22a-e) et de la tête de sonde (24a-e).

10. Dispositif de lithotripsie selon 7 ou 8, **caractérisé en ce que** dans la zone de mise en prise (26b : 26c) la tête de sonde (24a-e) entoure la tige de sonde (22a-e).

11. Dispositif de lithotripsie selon l'une quelconque des revendications 7 à 9, **caractérisé en ce que** dans la zone de mise en prise (26b,c), la tige de sonde (22a-e) et/ou la tête de sonde (24a-e) est/sont profilée(s).

12. Dispositif de lithotripsie selon l'une quelconque des revendications 7 à 9, **caractérisé en ce que** la tige de sonde (22a-e) et la tête de sonde (24a-e) sont pressées l'une contre l'autre, notamment serties.

13. Dispositif de lithotripsie selon l'une quelconque des revendications précédentes, **caractérisé en ce que** la tige de sonde (22a-e) et la tête de sonde (24a-e) sont reliées entre elles par une complémentarité de matière.

14. Dispositif de lithotripsie selon la revendication 13, **caractérisé en ce que** la tige de sonde (22a-e) et la tête de sonde (24a-e) sont surmoulées l'une avec l'autre.

15. Dispositif de lithotripsie selon l'une quelconque des revendications précédentes, **caractérisé en ce que** l'unité d'impulsion (14) comprend au moins une unité de choc (18), qui comprend au moins un projectile (20) monté de manière mobile pour fournir des impulsions de choc, qui est conçu, pour appliquer au moins indirectement des impulsions de choc à la sonde (12a-e).

16. Dispositif de lithotripsie selon la revendication 15, **caractérisé en ce que** le projectile (20) est au moins partiellement constitué du matériau amorphe (16).

17. Dispositif de lithotripsie selon l'une quelconque des revendications précédentes, **caractérisé en ce que** l'unité d'impulsion (14) comprend au moins une unité d'ultrasons (30), qui comprend au moins une corne à ultrasons (32) pour fournir et focaliser des impulsions ultrasonores, qui est conçue, pour appliquer au moins indirectement des impulsions ultrasonores à la sonde (12a-e).

18. Dispositif de lithotripsie selon la revendication 17, **caractérisé en ce que** la corne à ultrasons (32) est au moins partiellement constituée du matériau amorphe (16).

19. Lithotripteur (34) comprenant au moins un dispositif de lithotripsie selon l'une quelconque des revendications précédentes.

20. Procédé de fonctionnement non thérapeutique et non chirurgical d'un dispositif de lithotripsie selon l'une quelconque des revendications précédentes.
